(19) Europäisches Patentamt
European Patent Office
Office européen des brevets

(11) **EP 4 502 138 A1**

(12) **EUROPEAN PATENT APPLICATION**
published in accordance with Art. 153(4) EPC

(43) Date of publication:
**05.02.2025 Bulletin 2025/06**

(21) Application number: 23810584.5

(22) Date of filing: **03.03.2023**

(51) International Patent Classification (IPC):
*C12N 1/16* (2006.01)   *C12N 15/01* (2006.01)
*A21D 2/08* (2006.01)   *A21D 8/04* (2006.01)
*A21D 10/00* (2006.01)   *A21D 13/00* (2017.01)
*C12R 1/865* (2006.01)

(52) Cooperative Patent Classification (CPC):
**A21D 2/08; A21D 8/04; A21D 10/00; A21D 13/00;
C12N 1/16; C12N 15/01;** C12R 2001/865;
Y02E 50/10

(86) International application number:
**PCT/CN2023/079576**

(87) International publication number:
**WO 2023/226508 (30.11.2023 Gazette 2023/48)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 27.05.2022   CN 202210592941

(71) Applicant: **Angel Yeast Co., Ltd
Yichang, Hubei 443003 (CN)**

(72) Inventors:
• **SUN, Yafang
Yichang, Hubei 443003 (CN)**

• **ZHANG, Yan
Yichang, Hubei 443003 (CN)**
• **GUO, Tianfen
Yichang, Hubei 443003 (CN)**
• **KUANG, Jinbao
Yichang, Hubei 443003 (CN)**
• **CHEN, Liting
Yichang, Hubei 443003 (CN)**
• **YANG, Wenjing
Yichang, Hubei 443003 (CN)**

(74) Representative: **karo IP
Patentanwälte PartG mbB
Steinstraße 16-18
40212 Düsseldorf (DE)**

(54) **SACCHAROMYCES CEREVISIAE STRAIN, METHOD FOR SCREENING SAME, AND USE THEREOF**

(57)   The present invention relates to the technical field of microbial screening and food fermentation, and in particular, to a Saccharomyces cerevisiae strain, a method for screening same, and use thereof. The strain is Saccharomyces cerevisiae AMCC31194, which is deposited in the China Center for Type Culture Collection with deposite number CCTCC No.: M20211684. The strain of the present invention satisfies the urgent need for traits of wide sugar resistance, cold osmotic shock resistance, and organic acid resistance can be solved, and can improve the competitiveness of leading products, thereby fulfilling the market blank of calcium propionate-resistant yeast products for low-sugar dough fermentation.

EP 4 502 138 A1

## Description

## Technical Field

The present invention relates to the technical field of microbial screening and food fermentation, and in particular to a Saccharomyces cerevisiae strain, a screening method, and use thereof.

## Background Art

The proportion of low-sugar yeast used by baking industry users in the international market is very high, accounting for about 70% of the international market demand, mainly toast, hamburgers, hot dogs, and other bakery products with sugar content of about 5%. In particular, the Middle East region due to its geographical and climatic reasons, resulting in high baking ambient temperature, will cause the yeast will start too fast in the process of dough blending, resulting in the decline of product quality. Therefore, there is an urgent need for low-sugar yeast ice resistance in this region.

In addition, with the rapid development of the baking market, industrial users increasingly emphasize the scale, cost, product coverage, and delivery convenience, so the requirements for the shelf life of bakery products are also higher and higher. However, the use of "sodium dehydroacetate" was limited in the draft for comments of the revised edition of "National Food Safety Standards Standards for the Use of Food Additives", and the market demand for anti-corrosion of yeasts may change. At present, calcium propionate is the preservative meeting the latest standards for the use of food additives, so it is necessary to increase the research on the resistance of calcium propionate to yeast strains.

Patent CN1913780B discloses a bread yeast resistant to a high sugar concentration in the dough and to the presence of weak organic acids, optionally in the presence of mold inhibitors, fresh or dry Baker's yeast obtained from the strain, and their use in bread making, involving a sugar concentration in the range of 12-40%, weak organic acids (including calcium propionate) being present in the dough at a concentration of 0.4% in different sugar concentrations. Patent application CN102409002A discloses Saccharomyces cerevisiae strains suitable for the production of baker's yeasts which are osmotolerant and which exhibit intrinsic resistance to weak organic acids, methods for the preparation thereof and uses, involving an osmotic pressure resistance range of 15-25% and a weak organic acid intrinsic resistance concentration of 0.4%. Patent CN105829533 B discloses novel breadmaking yeast strains that are effective on non-sweetened or slightly sweetened doughs, the dough being a bread dough with a sugar concentration of less than 10%. Patent application CN113355251 A discloses a Saccharomyces cerevisiae strain with a certain degree of freezing resistance obtained by self-breeding, which can be effectively applied to different frozen dough products and involves a 0% sugar 1 h activity of 70 g dough. However, all the above-mentioned strains are obtained as mutant strains by hybridizing or mutation methods, and after screening of biomass and fermentation activity, yeast strains with superior dough fermentation activity under different systems are obtained, and the study on osmotic pressure and weak organic acid resistance mainly focuses on the presence of more than 12% sugar concentration and 0.4% calcium propionate, while the weak organic acid resistance is not studied under the condition of less than 10% sugar concentration, so the prior art lacks the study on the weak organic acid resistance at 0-12% sugar concentration, and in the practical baking application scenario, it is often necessary to consider the resistance of multiple indexes, such as the wide sugar resistance, the cold osmotic shock resistance, the organic acid resistance, etc.

It can be seen therefrom that in order to meet the practical application scenarios of bakery products and the need for preservation, yeast strains with sugar resistance, cold osmotic shock resistance, and organic acid resistance need to be developed.

## Summary of the Invention

In response to the problems of the prior art, the present invention provides a Saccharomyces cerevisiae strain having sugar resistance, cold osmotic shock resistance, and organic acid resistance, a method for screening same, and use thereof.

Specifically, the present invention proposes the following technical solutions.

The present invention provides a Saccharomyces cerevisiae strain, wherein the strain is Saccharomyces cerevisiae AMCC31194, which is deposited in the China Center for Type Culture Collection with deposite number CCTCC No.: M20211684.

Preferably, for a Saccharomyces cerevisiae strain as described above, wherein the Saccharomyces cerevisiae strain has a yeast milk dry weight of more than 90%, preferably 95-105% of that of the parent Saccharomyces cerevisiae strain.

Preferably, for a Saccharomyces cerevisiae strain as described above, wherein the Saccharomyces cerevisiae strain has a fermentation activity of more than 100% under a non-sweet dough system, preferably 110-125%, further preferably 120-125% of that of the parent Saccharomyces cerevisiae strain; and/or

the Saccharomyces cerevisiae strain has a fermentation activity of more than 100% under the medium-sugar dough system, preferably 105-120%, further preferably 110-120% of that of the parent Saccharomyces cerevisiae strain; and/or
the Saccharomyces cerevisiae strain has a fermentation activity of more than 100% under a medium-sugar dough system containing an organic acid or salt thereof, preferably 110-130%, further preferably 125-130% of that of the parent Saccharomyces cerevisiae strain.

Preferably, for a Saccharomyces cerevisiae strain as described above, wherein the active dry yeast of the Saccharomyces cerevisiae strain has a fermentation activity of more than 95% under a non-sweet dough system, preferably 100-110%, further preferably 104-110% of that of the parent Saccharomyces cerevisiae strain; and/or

the active dry yeast has a fermentation activity of more than 100% under the medium-sugar dough system, preferably 110-125%, further preferably 115-125% of that of the parent Saccharomyces cerevisiae strain; and/or
the active dry yeast has a fermentation activity of more than 100% under a medium-sugar dough system containing an organic acid or salt thereof, preferably 105-125%, further preferably 115-125% of that of the parent Saccharomyces cerevisiae strain.

The present invention provides a method for screening a Saccharomyces cerevisiae strain including:
forming sexual spores of a parent Saccharomyces cerevisiae strain and hybridizing single spores of different parent strains to obtain a hybrid strain having:
a yeast milk dry weight of the hybrid strain of more than 90% of that of the parent Saccharomyces cerevisiae strain.
Preferably, for the method described above, wherein the hybrid strain has:
a yeast milk dry weight of the hybrid strain of more than 95-105% of that of the parent Saccharomyces cerevisiae strain.
Preferably, for the method described above, wherein the hybrid strain has:

a fermentation activity of the hybrid strain of more than 100% under the non-sweet dough system, preferably 110-125%, further preferably 120-125% of that of the parent Saccharomyces cerevisiae strain; and/or
a fermentation activity of the hybrid strain of more than 100% under the medium-sugar dough system, preferably 105-120%, further preferably 110-120% of that of the parent Saccharomyces cerevisiae strain; and/or
a fermentation activity of the hybrid strain of more than 100% under a medium-sugar dough system containing an organic acid or salt thereof, preferably 110-130%, further preferably 125-130% of that of the parent Saccharomyces cerevisiae strain.

Preferably, for the method described above, wherein the hybrid strain has:

a fermentation activity of the active dry yeast of the hybrid strain of more than 95% under the non-sweet dough system, preferably 100-110%, further preferably 104-110% of that of the parent Saccharomyces cerevisiae strain; and/or
a fermentation activity of the active dry yeast of the hybrid strain of more than 100% under the medium-sugar dough system, preferably 110-125%, preferably 115-125% of that of the parent Saccharomyces cerevisiae strain; and/or
a fermentation activity of the active dry yeast of the hybrid strain of more than 100% under a medium-sugar dough system containing an organic acid or salt thereof, preferably 105-125%, preferably 115-125% of that of the parent Saccharomyces cerevisiae strain.

The present invention provides a Saccharomyces cerevisiae strain screened by the method described above, wherein the strain is Saccharomyces cerevisiae AMCC31194, which is deposited in the China Center for Type Culture Collection with deposite number CCTCC No.: M20211684. The present invention provides a fermentation composition including the Saccharomyces cerevisiae strain as described above or the Saccharomyces cerevisiae strain screened by a method as described above.
The present invention provides amicrobial inoculum including the Saccharomyces cerevisiae strain as described above or the Saccharomyces cerevisiae strain screened by a method as described above.
The present invention provides the use of the Saccharomyces cerevisiae strain as described above or the Saccharomyces cerevisiae strain screened by a method as described above or the fermentation composition as described above or the microbial inoculum as described above in food.
The present invention provides a dough including the Saccharomyces cerevisiae strain as described above or the Saccharomyces cerevisiae strain screened by a method as described above. The present invention provides a method for preparing the dough as described above including: fermenting the Saccharomyces cerevisiae strain as described above or the Saccharomyces cerevisiae strain screened by a method as described above to obtain a dough.

Preferably, for the method described above, wherein the Saccharomyces cerevisiae strain is fermented at 26-32°C.

The present invention provides a bakery product including the dough as described above or the dough prepared by a method as described above.

Preferably, for the bakery product as described above, wherein the bakery product is steamed bread, a steamed bun, or a bread stick.

Advantageous effects obtained by the present invention:

The strain described in the present invention is a wheat flour yeast strain, which can satisfy the urgent need for traits of wide sugar resistance, cold osmotic shock resistance, and organic acid resistance can be solved, and can improve the competitiveness of leading products, thereby fulfilling the market blank of calcium propionate-resistant yeast products for low-sugar dough fermentation.

**Strain deposit information**

The strain Saccharomyces cerevisiae AMCC 31194 of the present invention was deposited in the China Center for Type Culture Collection on December 29, 2021, with deposite number CCTCC NO: M20211684, deposited at Wuhan University, Wuhan, China, postal code: 430072, Tel: (027)68754562.

The strain Saccharomyces cerevisiae AMCC 30010 of the present invention was deposited in the China Center for Type Culture Collection on March 29, 2022, with deposite number CCTCC NO: M2022340, deposited at Wuhan University, Wuhan, China, postal code: 430072, Tel: (027)68754562.

The strain Saccharomyces cerevisiae AMCC 31195 of the present invention was deposited in the China Center for Type Culture Collection on December 29, 2021, with deposite number CCTCC NO: M20211685, deposited at Wuhan University, Wuhan, China, postal code: 430072, Tel: (027)68754562.

The strain Saccharomyces cerevisiae AMCC 30002 of the present invention was deposited in the China Center for Type Culture Collection (CCTCC) on March 29, 2022, with deposite number CCTCC NO: 2022338, deposited at Wuhan University, Wuhan, China, postal code: 430072, Tel: (027)-68754052.

The strain Saccharomyces cerevisiae AMCC 30004 of the present invention was deposited in the China Center for Type Culture Collection (CCTCC) on March 29, 2022, with deposite number CCTCC NO: 2022339, deposited at Wuhan University, Wuhan, China, postal code: 430072, Tel: (027)-68754052.

**Brief Description of the Drawings**

FIG. 1 is a schematic view of the electrophoresis results after performing the matching.
FIG. 2 is a schematic view of a sporulation test.

**Detailed Description of the Invention**

As described above, the present invention provides a Saccharomyces cerevisiae strain, wherein the strain is Saccharomyces cerevisiae AMCC31194, which is deposited in the China Center for Type Culture Collection with deposite number CCTCC No.: M20211684 (i.e. CCTCC M20211684).

The strain of the present invention is obtained by hybridizing and screening AMCC 31195 with deposite number CCTCC M 20211685 and AMCC 30010 (parent strain) with deposite number CCTCC M 2022340, the deposite number of the strain is CCTCC No.: M 20211684, the obtained strain has the properties of wide sugar resistance, cold osmotic shock resistance, and organic acid resistance, compared with the parent strain.

The wide sugar resistance means that the yeast strain has an advantage in fermentation activity in the range of 0-12% sugar.

The osmotic shock resistance means that the yeast still has an advantage in fermentation activity when the dough is kneaded with ice instead of water.

The AMCC 31195 strain with deposite number CCTCC M 20211685 is a Saccharomyces cerevisiae strain bred by AngelYeast Co., Ltd. and is a strain obtained by hybridizing the parent strains Saccharomyces cerevisiae AMCC 30002 strain and Saccharomyces cerevisiae AMCC 30004 strain, wherein the Saccharomyces cerevisiae AMCC30002 strain is a Saccharomyces cerevisiae strain bred by AngelYeast Co., Ltd. and the original strain is collected from Yantai City, Shandong Province. Through the observation of an optical microscope, the colony of Saccharomyces cerevisiae strain was cheese-like in texture, milky white in color, smooth in surface and neat in edge, oval in microscopic shape and budding. After 26S rDNA gene identification, the strain was identified as Saccharomyces cerevisiae strain which had a food attribute. It was deposited in the China Center for Type Culture Collection (CCTCC) on March 29, 2022, with deposite number CCTCC NO: M 2022338 (i.e. CCTCC M 2022338).

Saccharomyces cerevisiae AMCC30004 is a Saccharomyces cerevisiae strain bred by AngelYeast Co., Ltd. which was collected from the Kazakh Autonomous Prefecture of Ili in Xinjiang Uygur Autonomous Region. Through the observation of an optical microscope, the colony of Saccharomyces cerevisiae strain was cheese-like in texture, milky white in color, smooth in surface and neat in edge, oval in microscopic shape and budding. After 26S rDNA gene identification, the strain was identified as Saccharomyces cerevisiae strain which had a food attribute. It was deposited in the China Center for Type Culture Collection (CCTCC) on March 29, 2022, with deposite number CCTCC NO: M 2022339 (i.e. CCTCC M 2022339).

Hybridization method: The two parent strains sporulate under the same conditions, a single spore was inoculated into a test tube of YPD liquid culture medium, shaken at 30°C, and cultured overnight, then a hybridization combination was designed according to different matching, single spores of different matching were inoculated into a test tube of YPD liquid culture medium successively, shaken at 30°C, and cultured overnight, then diluted and spread onto a YPD plate, shaken at 30°C, and cultured overnight, and the growth of colonies was observed. The bigger colonies on the plate were selected and inoculated into the test tubes of YPD liquid culture medium, shaken at 30°C, and cultured overnight, the matching identification and sporulation test were performed on them, and the heterozygote was selected. The resulting heterozygotes were inoculated into 100-well plates containing high sugar culture medium, ready for on-machine determination by Bioscreen C instrument, and the parameters were set as follows: the growth curve determination was performed after measuring data every 30 min at a temperature of 30°C for 48 h at a wavelength of 600 nm. The maximum specific growth rate is analyzed according to the growth curve results, and the specific formula is as follows:

Maximum specific growth rate ($\mu$) = (lnOD2-lnOD1)/(t2-t1)
OD1: The corresponding $OD_{600}$ value at t1;
OD2: The corresponding $OD_{600}$ value at t2;
t2: End time of logarithmic growth phase;
t1: Start time of logarithmic growth phase;
according to the analysis of Bioscreen C high-throughput data, the first three heterozygous strains with the maximum specific growth rate were selected as one-generation strong heterozygotes.

According to the above steps, a single spore was prepared again, and the obtained single spore was hybridized with a single spore of the strain Saccharomyces cerevisiae AMCC 30004, heterozygote screening and heterozygote verification was performed successively, and the above steps were repeated to obtain a heterozygote of two rounds of hybridization, and the heterozygous strains with the first 100 maximum specific growth rate in the last round of heterozygotes were selected for a shake flask fermentation test.

The dry weight of yeast milk was used as the screening index, the heterozygous strains satisfying the dry weight index among the 100 heterozygous strains obtained above were screened. The shake flask experiment was performed on the above-mentioned heterozygous strains, the strains were inoculated into a YPD liquid culture medium shake flask, shaken at 30°C, and cultured overnight, to obtain yeast milk after centrifugation and washing, water content detection was performed, and dry weight of the yeast milk was calculated, wherein the screening criterion is that the dry weight of the yeast milk of the heterozygous strains reaches 95-100% of that of any parent strain;

the following tests were performed sequentially on heterozygous strains with dry weight advantage to screen heterozygous strains with high sugar resistance and organic acid resistance: Test A: Detection of the 1 h fermentation activity of 280 g dough under 16% sugar dough system, the screening criterion was 95-100% of that of any parent strain;
Test B: Detection of the 3 h fermentation activity of 70 g dough under 16% sugar + 1% calcium propionate dough system, the screening criterion was 95-100% of that of any parent strain, where Table 1 shows the formulas of dough systems for Test A and Test B.

Table 1 Formulas of dough systems for Test A and Test B

| Test raw material | A (g) | B (g) |
|---|---|---|
| Flour | 100 | 100 |
| Sucrose | 16 | 16 |
| Salt | 1.4 | 1.4 |

(continued)

| Test raw material | A (g) | B (g) |
|---|---|---|
| Dry yeast | 1 | 1 |
| Water | 45 | 45 |
| Calcium propionate | - | 1 |

The heterozygous strains with high sugar resistance and organic acid resistance obtained by screening were subjected to subsequent screening.

The hybrid strains with high sugar resistance and organic acid resistance were cultured in a small-scale fermenter. The yeast obtained from the fermenter was used to prepare the active dry yeast, which was used to prepare the dough containing 16% sugar + 0.6% calcium propionate, to screen heterozygous strains from which the active dry yeast prepared that had better fermentation performance in the environment of high sugar and organic acid. The screening criterion is that the 2 h fermentation activity of the heterozygous strain active dry yeast at 16% + 0.6% calcium propionate reached 100-120%, preferably 110-120% of that of any parent strain.

Finally, the cold osmotic shock resistance screening was performed on the heterozygous strains from which the active dry yeast prepared had better fermentation performance in the environment of high sugar and organic acid. 20% sugar dough containing hybrid strain active dry yeast was kneaded with crushed ice at 0°C and flour, and the leavening time of the dough was determined. The heterozygous strain with the shortest leavening time of the corresponding dough was used as the target strain to screen out the heterozygous strain with cold osmotic shock resistance.

a heterozygous strain with high sugar osmotic pressure resistance, cold osmotic shock resistance, and organic acid resistance was obtained by screening, and the heterozygous strain was identified, the obtained colony of Saccharomyces cerevisiae strain was cheese-like in texture, milky white in color, smooth in surface and neat in edge, oval in microscopic shape and budding.

The strain was named Saccharomyces cerevisiae AMCC 31195 strain, and the Saccharomyces cerevisiae AMCC 31195 strain was deposited at China Center for Type Culture Collection on December 29, 2021, with deposite number CCTCC NO: M 20211685 (i.e. CCTCC M 20211685). The AMCC 30010 with deposite number CCTCC M 2022340 is a Saccharomyces cerevisiae strain bred by AngelYeast Co., Ltd. The original strain was collected from Yichang City, Hubei Province. Through the observation of an optical microscope, the colony of Saccharomyces cerevisiae strain was cheese-like in texture, milky white in color, smooth in surface and neat in edge, oval in microscopic shape and budding. After 26S rDNA gene identification, the strain was identified as Saccharomyces cerevisiae strain which had a food attribute. The strain was deposited in the China Center for Type Culture Collection on March 29, 2022, with deposite number CCTCC NO: M2022340 (i.e. CCTCC M 2022340).

In some embodiments, the Saccharomyces cerevisiae strain has a yeast milk dry weight of more than 90%, preferably 95-105% of that of the parent Saccharomyces cerevisiae strain.

With regard to the method for determining the dry weight of the yeast milk, the present invention is not limited in any way, and it can be determined by a conventional method in the art; for example, the dry weight of the yeast milk can be determined by centrifuging the fermentation broth, collecting the yeast milk, weighing same, determining the water content thereof, and then performing conversion to obtain dry weight data.

The yeast milk dry weight of the Saccharomyces cerevisiae strain may be, for example, 90%, 91%, 92%, 93%, 94%, 95%, 96%, 97%, 98%, 99%, 100%, 101%, 102%, 103%, 104%, 105%, etc. of that of the parent Saccharomyces cerevisiae strain.

In some embodiments, the Saccharomyces cerevisiae strain has a fermentation activity of more than 100% under the non-sweet dough system, preferably 110-125%, further preferably 120-125% of that of the parent Saccharomyces cerevisiae strain; and/or

the Saccharomyces cerevisiae strain has a fermentation activity of more than 100% under the medium-sugar dough system, preferably 105-120%, further preferably 110-120% of that of the parent Saccharomyces cerevisiae strain; and/or
the Saccharomyces cerevisiae strain has a fermentation activity of more than 100% under a medium-sugar dough system containing an organic acid or salt thereof, preferably 110-130%, further preferably 125-130% of that of the parent Saccharomyces cerevisiae strain.

For example, the fermentation activity of the Saccharomyces cerevisiae strain under a non-sweet dough system can be 100%, 101%, 102%, 103%, 104%, 105%, 106%, 107%, 108%, 109%, 110%, 111%, 112%, 113%, 114%, 115%, 116%, 117%, 118%, 119%, 120%, 121%, 122%, 123%, 124%, 125%, etc. of that of the parent Saccharomyces cerevisiae strain;

the fermentation activity of the Saccharomyces cerevisiae strain under the medium-sugar dough system can be 100%, 101%, 102%, 103%, 104%, 105%, 106%, 107%, 108%, 109%, 110%, 111%, 112%, 113%, 114%, 115%, 116%, 117%, 118%, 119%, 120%, etc. of that of the parent Saccharomyces cerevisiae strain;

the fermentation activity of the Saccharomyces cerevisiae strain under a medium-sugar dough system containing an organic acid or salt thereof can be 100%, 101%, 102%, 103%, 104%, 105%, 106%, 107%, 108%, 109%, 110%, 111%, 112%, 113%, 114%, 115%, 116%, 117%, 118%, 119%, 120%, 121%, 122%, 123%, 124%, 125%, 126%, 127%, 128%, 129%, 130%, etc. of that of the parent Saccharomyces cerevisiae strain.

By dough it is meant that flour and other raw materials (liquids) are kneaded, by non-sweet dough it is meant dough that does not contain sugar, and by medium-sugar dough, it is meant dough that contains 7-12% sugar in the dough.

As the organic acid, the present invention is not limited in any way, and it may be an organic acid commonly used in the art, for example, propionic acid or acetic acid and the like, and as the salt of the organic acid, it may be a metal salt of the organic acid, for example, sodium propionate, calcium propionate, calcium acetate and the like.

In some embodiments, the active dry yeast of the Saccharomyces cerevisiae strain has a fermentation activity of more than 95% under the non-sweet dough system, preferably 100-110%, further preferably 104-110% of that of the parent Saccharomyces cerevisiae strain; and/or

the active dry yeast has a fermentation activity of more than 100% under the medium-sugar dough system, preferably 110-125%, further preferably 115-125% of that of the parent Saccharomyces cerevisiae strain; and/or

the active dry yeast has a fermentation activity of more than 100% under a medium-sugar dough system containing an organic acid or salt thereof, preferably 105-125%, further preferably 115-125% of that of the parent Saccharomyces cerevisiae strain.

The active dry yeast means that the strain is dried to obtain the active yeast. In the present invention, the present invention does not limit the drying method, and it can be selected according to the requirements, for example, the strain is cultured, separated, washed and dried, for example, the strain is cultured, separated, washed, suction filtered, pressure filtered, granulated, and packaged to obtain the active dry yeast.

For example, the fermentation activity of the active dry yeast of the Saccharomyces cerevisiae strain in the non-sweet dough system can be 95%, 96%, 97%, 98%, 99%, 100%, 101%, 102%, 103%, 104%, 105%, 106%, 107%, 108%, 109%, 110%, etc. of that of the parent Saccharomyces cerevisiae strain;

The fermentation activity of the active dry yeast under the medium-sugar dough system can be 100%, 101%, 102%, 103%, 104%, 105%, 106%, 107%, 108%, 109%, 110%, 111%, 112%, 113%, 114%, 115%, 116%, 117%, 118%, 119%, 120%, 121%, 122%, 123%, 124%, 125%, etc. of that of the parent Saccharomyces cerevisiae strain;

The fermentation activity of the active dry yeast in a medium-sugar dough system containing an organic acid or salt thereof can be 100%, 101%, 102%, 103%, 104%, 105%, 106%, 107%, 108%, 109%, 110%, 111%, 112%, 113%, 114%, 115%, 116%, 117%, 118%, 119%, 120%, 121%, 122%, 123%, 124%, 125%, etc. of that of the parent Saccharomyces cerevisiae strain.

The strains of the present invention have advantages in different application formulas (sugar-free formula, cold osmotic shock formula, medium-sugar formula, preserved formula, and full formula), indicating that the strains of the present invention have advantages in wide sugar resistance, preservative resistance, and cold osmotic shock resistance.

The present invention provides a method for screening a Saccharomyces cerevisiae strain including:

forming sexual spores of a parent Saccharomyces cerevisiae strain and hybridizing single spores of different parent strains to obtain a hybrid strain having:

a yeast milk dry weight of the hybrid strain of more than 90% of that of the parent Saccharomyces cerevisiae strain.

In some embodiments, the hybrid strain has: a yeast milk dry weight of the hybrid strain of more than 95-105% of that of the parent Saccharomyces cerevisiae strain. In some embodiments, the hybrid strain has: a fermentation activity of the hybrid strain of more than 100% under the non-sweet dough system, preferably 110-125%, further preferably 120-125% of that of the parent Saccharomyces cerevisiae strain; and/or a fermentation activity of the hybrid strain of more than 100% under the medium-sugar dough system, preferably 105-120%, further preferably 110-120% of that of the parent Saccharomyces cerevisiae strain; and/or a fermentation activity of the hybrid strain of more than 100% under a medium-sugar dough system containing an organic acid or salt thereof, preferably 110-130%, further preferably 125-130% of that of the parent Saccharomyces cerevisiae strain. In some embodiments, the hybrid strain has: a fermentation activity of the active dry yeast of the hybrid strain of more than 95% under the non-sweet dough system, preferably 100-110%, further preferably 104-110% of that of the parent Saccharomyces cerevisiae strain; and/or a fermentation activity of the active dry yeast of the hybrid strain of more than 100% under the medium-sugar dough system, preferably 110-125%, preferably 115-125% of that of the parent Saccharomyces cerevisiae strain; and/or a fermentation activity of the active dry yeast of the hybrid strain of more than 100% under a medium-sugar dough system containing an organic acid or salt thereof, preferably 105-125%, preferably 115-125% of that of the parent Saccharomyces cerevisiae strain.

The present invention provides a Saccharomyces cerevisiae strain screened by the method described above.

The present invention provides a fermentation composition including the Saccharomyces cerevisiae strain as described above or the Saccharomyces cerevisiae strain screened by a method as described above.

The present invention provides amicrobial inoculum including the Saccharomyces cerevisiae strain as described above or the Saccharomyces cerevisiae strain screened by a method as described above.

The present invention provides the use of the Saccharomyces cerevisiae strain as described above or the Saccharomyces cerevisiae strain screened by a method as described above or the fermentation composition as described above or the microbial inoculum as described above in food.

The present invention provides a dough including the Saccharomyces cerevisiae strain as described above or the Saccharomyces cerevisiae strain screened by a method as described above. With regard to the ratio of flour to other components such as water in the dough, the present invention is not limited in any way and may be conventionally selected as required, for example, the mass ratio of flour to water may be 100:50-55.

In the present invention, the present invention is not limited to the mass ratio of the dough and the strain, which may be conventionally selected as needed, for example, the mass ratio of the dough and the strain may be 100:0.5-5.

The present invention provides a method for preparing a dough as described above including: fermenting the Saccharomyces cerevisiae strain as described above or the Saccharomyces cerevisiae strain screened by a method as described above to obtain a dough. In some embodiments, the Saccharomyces cerevisiae strain is fermented at 26-32°C. As for the fermentation time, the present invention is not limited in any way, and it can be conventionally selected as required.

The present invention provides a bakery product including the dough as described above or the dough prepared by a method as described above. In some embodiments, the bakery product is steamed bread, a steamed bun, or a bread stick.

## Examples

The manufacturers of raw materials and equipment used in this example, as well as the equipment and analysis method used for product analysis, are described as follows. The chemical substances are not indicated as chemical purity grade of conventional reagents. Table 2 is the table of reagents and instruments used in the examples.

Table 2 Table of raw materials used in the examples

| Reagent/instrument | Model | Vendor |
|---|---|---|
| Yeast extract powder | - | Angel yeast |
| Glucose | - | SINOPHARM |
| Peptone | - | Angel yeast |
| Agar | - | HUIXING |
| Snail enzyme | - | Shanghai Sangong Biotech |
| 2 x PCR Mix | - | TIANGEN |
| pH meter | PB-10 | Sartorius |
| Rapid water comtent meter | MJ33 | METTLER TOLEDO |
| PCR instrument | C1000 | BIO-RAD |
| Gel imaging system | GelDoc™XR + | BIO-RAD |
| Electrophoresis apparatus | EPS-300 | Shanghai Tanon |
| Optical microscope | CX43 | OLYMPUS |
| Full-automatic growth curve analyzer | Bioscreen C | OY Growth Curves |
| Activity tester | SJA | SJA, Sweden |

Example 1 Screening of strains

(1) Analysis of the characteristics of parent strains

One strain of Saccharomyces cerevisiae (AMCC 31195 with deposite number CCTCC M 20211685) and one strain of Saccharomyces cerevisiae (AMCC 30010 with deposite number CCTCC M 2022340) were selected as starting test strains for hybridization feasibility analysis. Under the same conditions, both parent strains produced more than 90% spores, and the formula of sporulation rate was as follows.

The spore-forming colonies were dissolved with water, and snailase was added for enzymolysis, then the tetrad was observed by using a yeast micromanipulator, and single spores were separated, each single spore was placed on an agar plate at a precise position, the single spores of AMCC 31195 strain were successively named as S1-1 to S1-36 and the single spores of AMCC 30010 strain as S2-1 to S2-24, the genomes of the 60 single spores were extracted, and the genomes were extracted by using a yeast genome DNA extraction kit (model: DP307, manufacturer: TIANGEN BIOTECH CO.,LTD.); MAT-a (5'-ACTCCACTTCAAGTAAGAGTTTG-3', SEQ ID NO:1), MAT-$\alpha$ (5'-GCACGGAATATGGGAC-TACTTCG-3', SEQ ID NO:2), MAT-F (5'-AGTCACATCAAGATCGTTTATGG-3', SEQ ID NO:3) were used as primers, the amplification was performed by PCR instrument with PCR program of pre-denaturation at 94°C for 5 min, denaturation at 94°C for 30 s, annealing at 55°C for 30 s, extension at 72°C for 60 s for 30 cycles, and finally extension at 72°C for 10 min to amplify the yeast MATa/$\alpha$ gene sequence, which was detected by 1% gel electrophoresis apparatus, and the results are shown in Table 3 below:

$$Sporulation\ rate = \frac{Total\ number\ of\ spore-producing\ asci\ cells}{Total\ number\ of\ cells} \times 100\%$$

Note: Spore-producing asci cells include disporous, trisporous, and tetrasporous asci.

Table 3 Single spore survival rate and matching distribution of parent strains

| Strain | Tetrad number | Number of spores placed | Number of spores survived | Survival rate | Type MATa | Type MAT$\alpha$ |
|---|---|---|---|---|---|---|
| AMCC 31195 | 12 | 48 | 36 | 75.0% | 20 | 16 |
| AMCC 30010 | 10 | 40 | 24 | 60.0% | 14 | 10 |

As can be seen from Table 3, the parent strain AMCC 30010 has a relatively low single spore survival rate, may lack mating behavior during culture, and has hybridization limitations. In order to improve hybridization efficiency, the population hybridization method is selected for strain development.

(2) Strong spore screening and hybridization

The above-prepared single spore was inoculated into a test tube of YPD liquid culture medium (containing 1% yeast extract powder, 2% glucose, and 2% peptone), shaken at 30°C, cultured overnight, and then inoculated into a 100-well culture plate containing selection culture medium, ready for the on-machine determination by Bioscreen C instrument, and the parameters were set as follows: the growth curve determination was performed after measuring data every 30 min at a temperature of 30°C for 48 h at a wavelength of 600 nm. The specific growth rate is analyzed according to the growth curve results, and the growth rate calculation formula is calculated according to the above formula, namely:

$$\text{Maximum specific growth rate } (\mu) = (\ln OD2 - \ln OD1)/(t2 - t1)$$

OD1: The corresponding $OD_{600}$ value at t1;
OD2: The corresponding $OD_{600}$ value at t2;
t2: End time of logarithmic growth phase;
t1: Start time of logarithmic growth phase;

The single spores with the first three highest specific growth rates among the parent strains were selected, inoculated into test tubes of YPD liquid culture medium, shaken at 30°C, and cultured overnight, then inoculated into test tubes of YPD liquid medium according to the hybridization combination (Table 3), shaken at 30°C, and cultured overnight, then diluted and spread onto YPD plates, and cultured overnight at 30°C, and colony growth was observed. The maximum specific growth rate and matching of yeast single spores with growth advantage are shown in Table 4 and Table 5, respectively.

Table 4 Maximum specific growth rates and matching of yeast single spores with growth advantage

| Single spore number | Maximum specific growth rate $\mu$ ($h^{-1}$) | Matching of single spore |
|---|---|---|
| S1-8 | 0.0434 | MAT$\alpha$ |
| S1-16 | 0.0458 | MAT$\alpha$ |
| S1-30 | 0.0356 | MATa |
| S2-2 | 0.0381 | MATa |
| S2-7 | 0.0349 | MAT$\alpha$ |
| S2-15 | 0.0326 | MAT$\alpha$ |

Table 5 Hybrid combinations

| Combination number | Hybrid combination |
|---|---|
| I | S1-8($\alpha$) x S2-2(a) |
| II | S1-16($\alpha$) x S2-2(a) |
| III | S1-30(a) x S2-7($\alpha$) |
| IV | S1-30(a) x S2-15($\alpha$) |

(3) Heterozygote test and character screening

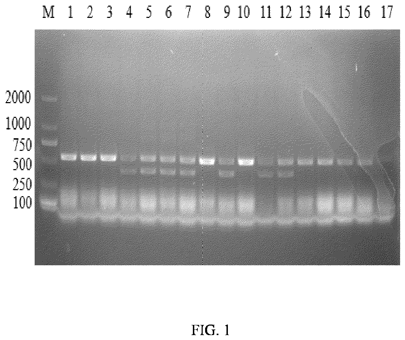

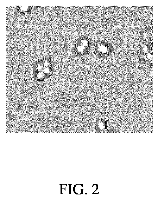

The colonies on the agar plate were inoculated into the test tubes of YPD liquid medium, shaken at 30°C, and cultured overnight, and then they were subjected to matching identification and sporulation tests (FIG. 1 and FIG. 2). The screening criterion was that the electrophoresis results were double bands and after sporulation, heterozygotes were identified as the ascospores were observed by optical microscope, and the wort culture medium screening was performed on the heterozygotes. The heterozygous strains with the first 60 maximum specific growth rates were selected for the shake flask fermentation test.

(4) Shake flask fermentation test

Shake flask tests were performed on the above-mentioned heterozygous strains, the strains were inoculated into YPD liquid culture medium shake flasks, shaken at 30°C, and cultured overnight, to obtain yeast milk after centrifugation and washing, and a rapid analyzer was used to perform water content detection, the dry weight (biomass is the weight of the yeast milk after centrifugation, dry weight (g/L) = biomass (g/L) * (1 - water content %)) and relative percentage (relative percentage = (dry weight of the heterozygous strains/dry weight of the parent strain) * 100%) were calculated, and the screening criterion was that the dry weight of the yeast milk of the hybrid strain reached more than 90% of that of any parent strain; preferably 95-105%, the strain was screened and named as AMCC 31194, and the relative percentages thereof were shown in Table 6. It can be seen from the results in Table 6 that the strain AMCC 31194 meets the preferred criteria and has been deposited in the China Center for Type Culture Collection, with deposite number CCTCC No: M20211684.

Table 6 Dry weight data (g/L) and relative percentage (%) of heterozygous strains

| Strain number | Dry weight | Relative percentage |
|---|---|---|
| AMCC 30010 | 13.23 | 100.0 |
| AMCC 31195 | 13.32 | 100.7 |
| AMCC 31194 | 12.76 | 96.4 |

The heterozygous strains with the dry weight advantage were detected for the fermentation activity of Tests A, B, and C in sequence. Test A: 0% sugar dough system; Test B: 12% sugar dough system; Test C: a 12% sugar + 1% calcium propionate dough system, wherein the component ratio of each baking raw material in the dough systems of Tests A, B and C (calculated by taking the mass of flour as 100%) was shown in table 7, and the weight of the yeast milk required was calculated; 1 h fermentation activity detection on 280 g of dough was performed in Test A, 1 h fermentation activity detection on 70 g of dough was performed in Test B, and 2 h fermentation activity detection on 70 g of dough was performed in Test C, wherein the fermentation activity was obtained by collecting the $CO_2$ gas produced by the dough using a SJA activity tester, and the relative fermentation activity was calculated; the calculation formula was relative fermentation activity = (fermentation activity of heterozygous strain/fermentation activity of parent strain) * 100%, i.e. the volume of $CO_2$ gas (mL) indicated the fermentation activity.

The screening criterion of test A is that the 1 h fermentation activity of the heterozygous strain yeast milk with 0% sugar was more than 100% preferably 110-125%, further preferably 120-125% of that of any parent strain; the screening criterion of test B is that the 1 h fermentation activity with 12% sugar is more than 100%, preferably 105-120%, further preferably 110-120% of that of any parent strain; the screening criterion of test C is that the 2 h fermentation activity with 12% sugar + 1% calcium propionate is more than 100%, preferably 110-130%, further preferably 125-130% of that of any parent strain. The results of AMCC 31194 are shown in Table 8. As can be seen from Table 8, the AMCC 31194 strain reached the preferred standard. Through the observation of the optical microscope on the AMCC31194 strain, the colony of Saccharomyces cerevisiae was cheese-like in texture, milky white in color, smooth in surface and neat in edge, oval in microscopic shape and budding. After 26S rDNA gene identification, the strain was identified as Saccharomyces cerevisiae strain.

Table 7 Ratio of baking raw materials for different dough systems (-mass%)

| Test / Raw material | A | B | C |
|---|---|---|---|
| Flour | 100 | 100 | 100 |
| Sugar | - | 12 | 12 |
| Salt | 1.4 | 1.4 | 1.4 |
| Dry yeast | 1 | 1 | 1 |
| Water | 50 | 50 | 50 |
| Calcium propionate | - | - | 1 |

Table 8 Fermentation activity (mL) and relative activity percentage (%) of heterozygous strains in Tests A, B and C

| Strain number | 1 h with 0% sugar | Relative activity | 1 h with 12% sugar | Relative activity | 2 h with 12% sugar + 1% calcium propionate | Relative activity |
|---|---|---|---|---|---|---|
| AMCC 30010 | 1050 | 100.0 | 79 | 100.0 | 55 | 100.0 |
| AMCC 31195 | 1174 | 111.8 | 125 | 158.2 | 123 | 223.6 |
| AMCC 31194 | 1284 | 122.3 | 90 | 113.9 | 69 | 126.2 |

(5) Lab-scale fermentation test

The preferred strains obtained by screening the shake flask fermentation test were cultured in a 30 L fermenter, separated, washed, suction filtered, pressure filtered, pelleted, dried, and packaged to obtain active dry yeast, and then the fermentation activity of 280 g dough was detected in tests A, B and D, wherein the addition amount of dry yeast in Test A was 1%, the system of Test B was the same as that of the shake flask fermentation test, and Test D: dough system containing 12% sugar + 0.6% calcium propionate, the baking raw material percentages were shown in Table 9, wherein test A detected 1 h fermentation activity, tests B and D detected 2 h fermentation activity, and the results were shown in Table 10.

Screening criteria for heterozygous strain dry yeast: 1 h fermentation activity with 0% sugar was more than 95%, preferably 100-110%, further preferably 104-110% of that of any parent strain, and 2 h fermentation activity with 12% sugar was more than 100%, preferably 110-125%, further preferably 115-125% of that of any parent strain; 2 h fermentation activity with 12% sugar + 0.6% calcium propionate reached more than 100%, preferably 105-125%, further preferably 115-125% of that of any parent strain. The results of the fermentation activity of AMCC 31194 are shown in Table 10. As can be seen from Table 10, the AMCC 31194 strain reached the preferred standard.

Table 9 Percentage of baking raw materials (-mass%) for dough system in test D

| Test / Raw material | D |
|---|---|
| Flour | 100 |
| Sugar | 12 |
| Salt | 1.4 |
| Dry yeast | 1 |
| Water | 50 |
| Calcium propionate | 0.6 |

Table 10 Percentage of relative activity of heterozygous strains in Tests A, B, D (%)

| Strain number | 1 h Fermentation activity with 0% sugar | Relative activity | 2 h Fermentation activity with 12% sugar | Relative activity | 2 h Fermentation activity with12% sugar + +0.6% calcium propionate | Relative activity |
|---|---|---|---|---|---|---|
| AMCC 30010 | 1016 | 100.0 | 1104 | 100.0 | 451 | 100.0 |
| AMCC 31195 | 742 | 73.0 | 1091 | 98.8 | 537 | 119.0 |
| AMCC 31194 | 1059 | 104.2 | 1304 | 118.1 | 528 | 117.0 |

Experimental examples

The present invention evaluated the AMCC 31194 strain on a 400g dough test with five application formulas: sugar-free formula, cold osmotic shock formula, medium-sugar formula, preservative formula, and full formula. The parent strain AMCC 30010 with more advantages in various traits was selected as the control strain. The fermentation time was taken as the index, the shorter the fermentation time indicated that the faster the dough rising speed under the same volume, the yeast strain had more advantages in traits, wherein the percentage of baking raw materials for each application formula was shown in Table 11 (crushed ice for the experiment was used in the cold osmotic shock resistance formula and the full formula, i.e. ice was added during dough kneading), and the relative percentage of fermentation time was shown in Table 12.

Table 11 Percentage of baking raw materials for application formula (-mass%)

| Raw material | Sugar-free formula (%) | Cold osmotic shock formula (%) | Medium-sugar formula (%) | Preservative formula (%) | Full formula (%) |
|---|---|---|---|---|---|
| Flour | 100 | 100 | 100 | 100 | 100 |
| Yeast | 1 | 1 | 1 | 1 | 1 |
| Common salt | 2 | 2 | 1 | 1 | 1 |
| Sugar | / | / | 12 | 12 | 12 |
| Calcium propionate | / | / | / | 0.6 | 0.6 |
| Water | 65 | 63 (ice) | 60 | 60 | 63 (ice) |

Table 12 Relative percentage of fermentation time for AMCC 31194 strain application test (%)

| Formula | Percentage of fermentation time relative to control (%) |
|---|---|
| Sugar-free | 97.0 |
| Cold osmotic shock | 92.6 |
| Medium-sugar | 94.0 |

(continued)

| Formula | Percentage of fermentation time relative to control (%) |
|---------|----------------------------------------------------------|
| Preservative | 93.3 |
| Full formula | 86.8 |

The results showed that compared with the control strain, the AMCC 31194 strain had an obvious advantage in fermentation time in each application formula, especially in the full formula which had the advantage of about 13%. The application tests further showed that the AMCC 31194 strain had the advantage of wide sugar resistance, preservative resistance, and cold osmotic shock resistance.

The foregoing is illustrative of the preferred embodiments of the present invention and is not to be construed as limiting the invention in any way. Thus, it is intended that the present invention cover the modifications, equivalents, and alternatives falling within the spirit and scope of the invention.

**Claims**

1.  A Saccharomyces cerevisiae strain, **characterized in that** the strain is Saccharomyces cerevisiae AMCC31194, which is deposited in the China Center for Type Culture Collection with deposite number CCTCC No.: M20211684.

2.  The Saccharomyces cerevisiae strain according to claim 1, **characterized in that** the Saccharomyces cerevisiae strain has a yeast milk dry weight of more than 90%, preferably 95-105% of that of a parent Saccharomyces cerevisiae strain.

3.  The Saccharomyces cerevisiae strain according to claim 2, **characterized in that** the Saccharomyces cerevisiae strain has a fermentation activity of more than 100% under a non-sweet dough system, preferably 110-125%, further preferably 120-125% of that of the parent Saccharomyces cerevisiae strain; and/or

    the Saccharomyces cerevisiae strain has a fermentation activity of more than 100% under the medium-sugar dough system, preferably 105-120%, further preferably 110-120% of that of the parent Saccharomyces cerevisiae strain; and/or
    the Saccharomyces cerevisiae strain has a fermentation activity of more than 100% under a medium-sugar dough system containing an organic acid or salt thereof, preferably 110-130%, further preferably 125-130% of that of the parent Saccharomyces cerevisiae strain.

4.  The Saccharomyces cerevisiae strain according to claim 2 or 3, **characterized in that** an active dry yeast of the Saccharomyces cerevisiae has a fermentation activity of more than 95% under the non-sweet dough system, preferably 100-110%, further preferably 104-110% of that of the parent Saccharomyces cerevisiae strain; and/or

    the active dry yeast has a fermentation activity of more than 100% under the medium-sugar dough system, preferably 110-125%, further preferably 115-125% of that of the parent Saccharomyces cerevisiae strain; and/or
    the active dry yeast has a fermentation activity of more than 100% under a medium-sugar dough system containing an organic acid or salt thereof, preferably 105-125%, further preferably 115-125% of that of the parent Saccharomyces cerevisiae strain.

5.  A method for screening a Saccharomyces cerevisiae strain, comprising:
    forming sexual spores of a parent Saccharomyces cerevisiae strain and hybridizing single spores of different parent strains to obtain a hybrid strain having:
    a yeast milk dry weight of the hybrid strain of more than 90% of that of the parent Saccharomyces cerevisiae strain.

6.  The method according to claim 5, **characterized in that** the hybrid strain has:
    a yeast milk dry weight of the hybrid strain of more than 95-105% of that of the parent Saccharomyces cerevisiae strain.

7.  The method according to claim 5 or 6, **characterized in that** the hybrid strain has:

    a fermentation activity of the hybrid strain of more than 100% under the non-sweet dough system, preferably 110-125%, further preferably 120-125% of that of the parent Saccharomyces cerevisiae strain; and/or
a fermentation activity of the hybrid strain of more than 100% under the medium-sugar dough system, preferably 105-120%, further preferably 110-120% of that of the parent Saccharomyces cerevisiae strain; and/or
a fermentation activity of the hybrid strain of more than 100% under a medium-sugar dough system containing an organic acid or salt thereof, preferably 110-130%, further preferably 125-130% of that of the parent Saccharomyces cerevisiae strain.

8. The method according to any one of claims 5 to 7, **characterized in that** the hybrid strain has:

a fermentation activity of the active dry yeast of the hybrid strain of more than 95% under the non-sweet dough system, preferably 100-110%, further preferably 104-110% of that of the parent Saccharomyces cerevisiae strain; and/or
a fermentation activity of the active dry yeast of the hybrid strain of more than 100% under the medium-sugar dough system, preferably 110-125%, preferably 115-125% of that of the parent Saccharomyces cerevisiae strain; and/or
a fermentation activity of the active dry yeast of the hybrid strain of more than 100% under a medium-sugar dough system containing an organic acid or salt thereof, preferably 105-125%, preferably 115-125% of that of the parent Saccharomyces cerevisiae strain.

9. A Saccharomyces cerevisiae strain screened by the method according to any one of claims 5 to 8, **characterized in that** the strain is Saccharomyces cerevisiae AMCC31194, which is deposited in the China Center for Type Culture Collection with deposite number CCTCC No.: M20211684.

10. A fermentation composition, **characterized by** comprising the Saccharomyces cerevisiae strain according to any one of claims 1 to 4 or the Saccharomyces cerevisiae strain screened by the method according to any one of claims 4 to 8.

11. A microbial inoculum, **characterized by** comprising the Saccharomyces cerevisiae strain according to any one of claims 1 to 4 or the Saccharomyces cerevisiae strain screened by the method according to any one of claims 4 to 8.

12. Use of the Saccharomyces cerevisiae strain according to any one of claims 1 to 4 or the Saccharomyces cerevisiae strain screened by the method according to any one of claims 4 to 8 or the fermentation composition according to claim 10 or the microbial inoculum according to claim 11 in food.

13. A dough, **characterized by** comprising the Saccharomyces cerevisiae strain according to any one of claims 1 to 4 or the Saccharomyces cerevisiae strain screened by the method according to any one of claims 4 to 8.

14. A method for preparing the dough according to claim 13, **characterized by** comprising:
fermenting the Saccharomyces cerevisiae strain according to any one of claims 1 to 4 or the Saccharomyces cerevisiae strain screened by the method according to any one of claims 4 to 8 to obtain a dough.

15. The method according to claim 14, **characterized in that** the Saccharomyces cerevisiae strain is fermented at 26-32°C.

16. A bakery product, **characterized by** comprising the dough according to claim 13 or a dough prepared by the method according to any one of claims 14 and 15.

17. The bakery product according to claim 16, **characterized in that** the bakery product is steamed bread, a steamed bun, or a bread stick.

FIG. 1

FIG. 2

## INTERNATIONAL SEARCH REPORT

| International application No. |
| --- |
| **PCT/CN2023/079576** |

| | |
| --- | --- |
| **A.** | **CLASSIFICATION OF SUBJECT MATTER** |
| | C12N 1/16(2006.01)i; C12N15/01(2006.01)i; A21D2/08(2006.01)i; A21D8/04(2006.01)i; A21D10/00(2006.01)i; A21D13/00(2017.01)i; C12R1/865(2006.01)i |
| | According to International Patent Classification (IPC) or to both national classification and IPC |

| | |
| --- | --- |
| **B.** | **FIELDS SEARCHED** |

Minimum documentation searched (classification system followed by classification symbols)

IPC: C12N, A21D, C12R

Documentation searched other than minimum documentation to the extent that such documents are included in the fields searched

Electronic data base consulted during the international search (name of data base and, where practicable, search terms used)

CNTXT, WPABS, WPABSC, OETXT, USTXTC, ENTXT, ENTXTC, VEN, DWPI, CJFD; CNKI, 万方数据WANFANG Data, 超星读秀, DUXIU, PubMed, Elsevier Science, ISI WEB of Science: 酿酒酵母, 酵母乳, 杂交, 杂合, 孢, 不甜, 微甜, 中糖, 有机酸, 丙酸, 乙酸, 冷, 冰, Saccharomyces, cerevisiae, Yeast milk, hybrid, heterozygous, spore, unsweet, slightly sweet, Middle sugar, organic acid, Calcium propionate, calcium acetate, cold, ice

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| X | CN 101821380 A (LESAFFRE ET COMPAGNIE) 01 September 2010 (2010-09-01) see claims 1-12, and description, paragraphs 8, 11, 16, 19, 54, 116, 164-188 and 196-204, and embodiment 2, and tables 3-4 | 1-17 |
| X | JP H0670673 A (NATIONAL FOOD RESEARCH INSTITUTE; ASAHI CHEMICAL INDUSTRY CO., LTD.) 15 March 1994 (1994-03-15) see claims 1-3, and description, paragraphs 12-15, and embodiments, and tables 4-5 | 1-17 |
| X | CN 113355251 A (ANGEL YEAST CO., LTD.) 07 September 2021 (2021-09-07) see abstract, and description, paragraph 34, and embodiments 1-2 and 4, and tables 2, 5 and 9 | 5-8, 10-17 |
| X | US 2021186038 A1 (PAK GIDA URETIM VE PAZARLAMA A.S.) 24 June 2021 (2021-06-24) see claims 1-12 | 5-8, 10-17 |
| A | CN 101171936 A (JAPAN TOBACCO INC.) 07 May 2008 (2008-05-07) see claims 1-11 | 1-17 |

| | |
| --- | --- |
| ✓ Further documents are listed in the continuation of Box C. | ✓ See patent family annex. |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **30 May 2023** | **11 June 2023** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/079576**

**C.    DOCUMENTS CONSIDERED TO BE RELEVANT**

| Category* | Citation of document, with indication, where appropriate, of the relevant passages | Relevant to claim No. |
|---|---|---|
| A | CN 1913780 A (LESAFFRE ET COMPAGNIE) 14 February 2007 (2007-02-14)<br>see claims 1-16, and description, pp. 3-4 | 1-17 |

| INTERNATIONAL SEARCH REPORT | International application No. |
|---|---|
| Information on patent family members | PCT/CN2023/079576 |

| Patent document cited in search report | | | Publication date (day/month/year) | Patent family member(s) | | | Publication date (day/month/year) |
|---|---|---|---|---|---|---|---|
| CN | 101821380 | A | 01 September 2010 | EP | 2183354 | A1 | 12 May 2010 |
| | | | | US | 2011129567 | A1 | 02 June 2011 |
| | | | | FR | 2920157 | A1 | 27 February 2009 |
| | | | | FR | 2920157 | B1 | 16 October 2009 |
| | | | | RU | 2010105632 | A | 27 September 2011 |
| | | | | MX | 2010002089 | A | 04 August 2010 |
| | | | | WO | 2009056708 | A1 | 07 May 2009 |
| JP | H0670673 | A | 15 March 1994 | JPH | 0771444 | B2 | 02 August 1995 |
| CN | 113355251 | A | 07 September 2021 | | None | | |
| US | 2021186038 | A1 | 24 June 2021 | WO | 2020145930 | A2 | 16 July 2020 |
| | | | | WO | 2020145930 | A3 | 27 August 2020 |
| | | | | TR | 201900376 | A2 | 21 February 2019 |
| CN | 101171936 | A | 07 May 2008 | JP | 2004033207 | A | 05 February 2004 |
| | | | | JP | 4226890 | B2 | 18 February 2009 |
| | | | | US | 2006134268 | A1 | 22 June 2006 |
| | | | | EP | 1541671 | A1 | 15 June 2005 |
| | | | | EP | 1541671 | A4 | 30 August 2006 |
| | | | | EP | 1541671 | B1 | 05 November 2008 |
| | | | | KR | 20050016710 | A | 21 February 2005 |
| | | | | KR | 100858177 | B1 | 10 September 2008 |
| | | | | TW | 200401026 | A | 16 January 2004 |
| | | | | TWI | 330665 | B | 21 September 2010 |
| | | | | WO | 2004005490 | A1 | 15 January 2004 |
| | | | | JPWO | 2004005490 | A1 | 04 November 2005 |
| | | | | JP | 4014167 | B2 | 28 November 2007 |
| | | | | AU | 2002318623 | A1 | 23 January 2004 |
| CN | 1913780 | A | 14 February 2007 | EP | 1559322 | A1 | 03 August 2005 |
| | | | | EP | 1559322 | B1 | 23 July 2008 |
| | | | | DK | 1559322 | T3 | 24 November 2008 |
| | | | | US | 2007122524 | A1 | 31 May 2007 |
| | | | | ES | 2308053 | T3 | 01 December 2008 |
| | | | | AT | 401792 | T | 15 August 2008 |
| | | | | JP | 2007519412 | A | 19 July 2007 |
| | | | | JP | 4749341 | B2 | 17 August 2011 |
| | | | | EP | 1705994 | A1 | 04 October 2006 |
| | | | | DE | 602004015239 | D1 | 04 September 2008 |
| | | | | MXPA | 06008580 | A | 15 March 2007 |
| | | | | BRPI | 0507142 | A | 19 June 2007 |
| | | | | CA | 2553596 | A1 | 22 September 2005 |
| | | | | WO | 2005087012 | A1 | 22 September 2005 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1913780 B
- CN 102409002 A
- CN 105829533 B
- CN 113355251 A